# EUROPÄISCHE PATENTSCHRIFT

(11) **EP 4 067 332 B1**
(45) Veröffentlichungstag und Bekanntmachung des Hinweises auf die Patenterteilung: **18.06.2025**
(21) Anmeldenummer: 22157463.5
(22) Anmeldetag: 18.02.2022
(51) Int. Cl.: C07C 51/41, C07C 53/12

(54) **VERFAHREN ZUR HERSTELLUNG VON ALUMINIUMSALZEN EINER FETTSÄURE**
METHOD FOR THE PREPARATION OF ALUMINIUM SALTS OF A FATTY ACID
PROCÉDÉ DE PRODUCTION DE SELS D'ALUMINIUM D'UN ACIDE GRAS

(30) Priorität: 19.02.2021 EP 21158204
(43) Veröffentlichungstag der Anmeldung: 05.10.2022
(73) Patentinhaber: Peter Greven GmbH & Co. KG, 53902 Bad Münstereifel (DE)
(72) Erfinder: STOLZ, Hermann Josef, 53902 Bad Münstereifel (DE); HUBER, Wilhelm, 50169 Kerpen (DE); KEHREN, Gabriel, 53879 Euskirchen (DE)
(74) Vertreter: dompatent von Kreisler Selting Werner - Partnerschaft von Patent- und Rechtsanwälten mbB

(56) Entgegenhaltungen:
- EVA LONCAR ET AL: "Preparation and characterization of aluminum stearate", ACTA PERIODICA TECHNOLOGICA, 1 January 2003 (2003-01-01), pages 55 - 60, XP055333402, Retrieved from the Internet <URL:http://www.doiserbia.nb.rs/img/doi/1450-7188/2003/1450-71880334055L.pdf> DOI: 10.2298/APT0334055L

## Beschreibung

Die vorliegende Erfindung betrifft ein Verfahren zur Herstellung von Aluminiumsalzen einer Fettsäure sowie Aluminiumsalze einer Fettsäure erhältlich nach einem solchen Verfahren.

Aluminiumsalze von Fettsäuren werden auch als Aluminiumseifen bezeichnet. Sie besitzen hydrophobe Eigenschaften und zeichnen sich durch eine wachsartige Konsistenz aus. Aufgrund der Dreiwertigkeit der enthaltenen Aluminiumionen können Aluminiumsalze von Fettsäuren als Mono-, Di- oder Trifettsäurealuminiumsalze vorliegen. Die Mono- oder Difettsäurealuminiumsalze können neben den Fettsäure-Anionen als zusätzliche Gegenionen für das dreiwertige Aluminiumion Hydroxidionen enthalten. Die in den Mono- oder Difettsäurealuminiumsalzen enthaltenen Hydroxidionen verleihen den Aluminiumsalzen der Fettsäure dispergierende Eigenschaften. Dadurch sind die Aluminiumsalze von Fettsäuren zur Verdickung von Ölen geeignet und können in der pharmazeutischen Industrie oder der Kosmetikindustrie beispielsweise zur Herstellung von Creme- oder Salbengrundlagen verwendet werden.

Die industrielle Herstellung von Aluminiumsalzen von Fettsäuren erfolgt vorwiegend nach dem Fällverfahren ("doppelte Umsetzung"). Hierbei wird in einem ersten Reaktionsschritt zunächst in wässriger Lösung ein Natriumsalz einer Fettsäure gebildet. In einem zweiten Reaktionsschritt wird das Aluminiumsalz der Fettsäure durch Zugabe von Aluminiumsulfat oder -chlorid gefällt. Ein Nachteil dieses Verfahrens ist, dass die als Nebenprodukte entstehenden Salze Natriumsulfat oder -chlorid sich nicht leicht von dem Aluminiumsalz der Fettsäure abtrennen lassen. Ein vollständiges oder nahezu vollständiges Auswaschen der Nebenprodukte ist nur mit großem Aufwand zu realisieren.

Dies ist insbesondere in Bezug auf eine Anwendung der Aluminiumsalze von Fettsäuren in der pharmazeutischen Industrie von Nachteil. Aluminiumsalze von Fettsäuren, beispielsweise Aluminiumstearate, werden in der pharmazeutischen Industrie zum Beispiel bei der Herstellung von Kapseln, Inhalationssprays oder Salben verwendet. Gemäß Europäischem Arzneibuch *(Pharmacopoea Europaea,* Ph. Eur.) ist beispielsweise der Gehalt an Chlorid in Aluminiumstearat auf 1000 ppm, der Gehalt an Sulfat auf 5000 ppm begrenzt. Da sich die beim zuvor beschriebenen Fällverfahren entstehenden Nebenprodukte Natriumsulfat und Natriumchlorid nur unter großem Aufwand aus dem Produkt entfernen lassen, lassen sich die geforderten geringen Chlorid- und Sulfatgehalte nicht wirtschaftlich realisieren.

Loncar et al., APTEFF34(2003) 1-148 beschreibt die Herstellung und Charakterisierung von Aluminiumstearaten.

US 2 469 041 beschreibt ein weiteres Verfahren zur Herstellung von Aluminiumsalzen von Fettsäuren. Hierbei werden zunächst die Aluminiumalkoholate niedermolekularer Alkohole hergestellt. Diese werden in einer Ölmatrix direkt mit den gewünschten Fettsäuren zur Reaktion gebracht. Die Nebenprodukte der Reaktion - niedermolekulare Alkohole - werden verdampft. Von Nachteil ist jedoch, dass sich das beschriebene Verfahren nur zur *in* situ-Herstellung von Schmierfetten eignet.

Es ist daher die Aufgabe der vorliegenden Erfindung, ein Verfahren zur Herstellung von Aluminiumsalzen einer Fettsäure zur Verfügung zu stellen, welches zumindest einige der aus dem Stand der Technik bekannten Probleme überwindet.

Gelöst wird die erfindungsgemäße Aufgabe durch ein Verfahren zur Herstellung von Aluminiumsalzen einer Fettsäure, umfassend die folgenden Schritte:
a) Vermischen einer Fettsäure und einer wässrigen Lösung einer starken Base zur Herstellung einer wässrigen Base-Fettsäure-Mischung,
b) Vermischen der wässrigen Base-Fettsäure-Mischung mit einer Aluminiumquelle zur Herstellung einer wässrigen Base-Fettsäure-Aluminium-Mischung,
c) Vermischen der wässrigen Base-Fettsäure-Aluminium-Mischung mit einer Säure, und
d) Abtrennung der entstandenen Aluminiumsalze der Fettsäure.

In Schritt a) des erfindungsgemäßen Verfahrens zur Herstellung von Aluminiumsalzen einer Fettsäure wird eine wässrige Base-Fettsäure-Mischung hergestellt, indem eine starke Base mit Wasser vermischt wird. Dadurch wird eine wässrige Lösung einer starken Base erhalten. Anschließend wird eine Fettsäure zu der zuvor gebildeten wässrigen Lösung der starken Base gegeben. Die Fettsäure wird in der wässrigen Base-Lösung gelöst. Bevorzugt umfasst die wässrige Base-Fettsäure-Mischung aus Schritt a) eine deprotonierte Fettsäure.

In Schritt b) des erfindungsgemäßen Verfahrens zur Herstellung von Aluminiumsalzen einer Fettsäure wird eine wässrige Base-Fettsäure-Aluminium-Mischung hergestellt, indem eine Aluminiumquelle zu der wässrigen Base-Fettsäure-Mischung aus Schritt a) zugegeben und mit dieser vermischt wird. Bevorzugt wird die Aluminiumquelle in der wässrigen Base-Fettsäure-Mischung aus Schritt a) gelöst. Bevorzugt handelt es sich bei der Aluminiumquelle aus Schritt b) um eine wässrige Lösung der Aluminiumquelle.

In Schritt c) des erfindungsgemäßen Verfahrens zur Herstellung von Aluminiumsalzen einer Fettsäure wird die wässrige Base-Fettsäure-Aluminium-Mischung aus Schritt b) mit einer Säure versetzt. Die Säure wird mit der wässrigen Base-Fettsäure-Aluminium-Mischung vermischt.

In Schritt d) des erfindungsgemäßen Verfahrens zur Herstellung von Aluminiumsalzen einer Fettsäure werden die entstandenen Aluminiumsalze der Fettsäure von den übrigen Bestandteilen der angesäuerten wässrigen Base-Fettsäure-Aluminium-Mischung aus Schritt c) abgetrennt. Die Trennung der Aluminiumsalze der Fettsäure von den übrigen Bestandteilen der angesäuerten wässrigen Base-Fettsäure-Aluminium-Mischung kann durch ein physikalisches Trennverfahren wie Filtration oder Zentrifugation erfolgen. Bevorzugt erfolgt die Trennung durch Filtration. Die Aluminiumsalze der Fettsäure stellen hierbei den Filterkuchen dar. Zur Verbesserung der Trennung der entstandenen Aluminiumsalze der Fettsäure von den übrigen Bestandteilen kann der Filterkuchen mit Wasser, bevorzugt mit destilliertem Wasser, gewaschen werden. Bei der Abtrennung mittels Zentrifugation bilden die Aluminiumsalze der Fettsäure das Präzipitat.

Bei den mit dem erfindungsgemäßen Verfahren hergestellten Aluminiumsalzen einer Fettsäure kann es sich um Mono-, Di, oder Trifettsäurealuminiumsalze handeln.

Die in dem erfindungsgemäßen Verfahren zur Herstellung von Aluminiumsalzen einer Fettsäure verwendeten Fettsäuren stammen bevorzugt aus natürlichen Quellen. Die Fettsäuren können beispielsweise aus Palm- oder Palmkernöl, Kokosfett, Talg, Rizinusöl, aber auch aus Raps oder Soja gewonnen werden. Es kann sich um gesättigte oder ungesättigte Fettsäuren handeln.

Bevorzugt handelt es sich bei der im erfindungsgemäßen Verfahren verwendeten Fettsäuren um Fettsäure der Kettenlängen C6 bis C28 oder Mischungen davon. Mehr bevorzugt ist die Fettsäure ausgewählt aus der Gruppe bestehend aus Stearinsäure, Palmitinsäure und Mischungen dieser Fettsäuren.

In einer Ausführungsform des erfindungsgemäßen Verfahrens handelt es sich bei der Fettsäure um Stearinsäure.

Kommerziell erhältliche Stearinsäure wird zum überwiegenden Teil aus Talg oder Palmöl gewonnen. Entsprechend ihrer Herkunft besteht die kommerziell erhältliche Stearinsäure üblicherweise nicht aus reiner Stearinsäure (Octadecansäure), sondern weist eine, für ihre Herkunft typische, Fettsäureverteilung auf. Genaugenommen handelt es sich also bei kommerziell erhältlicher Stearinsäure häufig um eine Fettsäuremischung, welche Stearinsäure umfasst. Wird die Stearinsäure aus Talg gewonnen, enthält sie etwa 65 Gew.-% Octadecansäure und 25 Gew.-% Palmitinsäure (Hexadecansäure). Wird die Stearinsäure aus Palmöl oder Palmkernöl gewonnen, so kann sie über einen Gehalt von 50 Gew.-% oder 65 Gew.-% Octadecansäure verfügen. Am Markt erhältliche Stearinsäure kann auch deutlich weniger als 50 Gew.-% Octadecansäure enthalten. Der Gehalt an Hexadecansäure ist dementsprechend höher.

In einer Ausführungsform des erfindungsgemäßen Verfahrens wird als Fettsäure aus Talg gewonnene Stearinsäure mit einem Gehalt an Octadecansäure von 50 bis 70 Gew.-% verwendet. In einer weiteren Ausführungsform wird aus Palmöl gewonnene Stearinsäure mit einem Gehalt an Octadecansäure von 40 bis 70 Gew.-%% verwendet.

In einer weiteren Ausführungsform wird Stearinsäure in einer Reinheit von >80 Gew.-% verwendet. Es kann auch Stearinsäure mit einer Reinheit von ≥98 Gew.-% verwendet werden.

Bevorzugt handelt es sich bei der starken Base aus Schritt a) um ein Alkalihydroxid. Mehr bevorzugt handelt es sich um Natriumhydroxid oder Kaliumhydroxid. Besonders bevorzugt handelt es sich bei der starken Base um Natriumhydroxid. Die Konzentration der starken Base in der durch Mischung der starken Base mit Wasser erhaltenen wässrigen Lösung der starken Base liegt bevorzugt bei 0,2 mol/L oder mehr. Die Konzentration liegt bevorzugt bei 1,0 mol/L oder weniger. Mehr bevorzugt liegt die Konzentration zwischen 0,3 mol/L und 0,8 mol/L, besonders bevorzugt zwischen 0,35 mol/L und 0,75 mol/L.

Der pH-Wert der wässrigen Base-Fettsäure-Mischung aus Schritt a) liegt bevorzugt im Bereich zwischen 7,0 und 12,0, mehr bevorzugt zwischen 8,0 und 11,0.

Bevorzugt handelt es sich bei der in Schritt b) verwendeten Aluminiumquelle um ein Alkalialuminat oder um Aluminiumpulver. Mehr bevorzugt handelt es sich bei der Aluminiumquelle um Natriumaluminat oder Aluminiumpulver. Noch mehr bevorzugt handelt es sich bei der Aluminiumquelle um Natriumaluminat. Alkalialuminate sind Salze der Aluminiumsäure HAlO₂ × H₂O, bei denen Aluminium ein komplexes Anion [Al(OH)₄]⁻ mit Hydroxidion als Liganden bildet, sowie Salze, bei denen das Anion als Kondensat des Aluminat-Ions vorliegt. Die allgemeine Zusammensetzung solcher Verbindungen ist M[Al(OH)_{4]}, wobei M ein Alkalikation, bevorzugt Na⁺, ist. Vollständig kondensierte, wasserfreie Verbindungen haben die allgemeine Zusammensetzung MAlO₂ mit AlO₂⁻ als Anion, wobei M ein Alkalikation, bevorzugt Na⁺, ist. In Schritt b) des erfindungsgemäßen Verfahrens kann kommerziell erhältliches NaAlO₂ als Aluminiumquelle eingesetzt werden.

Bevorzugt beträgt die Konzentration der Aluminiumquelle in der wässrigen Base-Fettsäure-Aluminium-Mischung in Schritt b) zwischen 50 mmol/L und 600 mmol/L, mehr bevorzugt zwischen 100 mmol/L und 500 mmol/L, jeweils gemessen als Aluminium. Verfahren zur quantitativen Bestimmung von Aluminium sind dem Fachmann bekannt. Der Gehalt an Aluminium kann beispielsweise mittels Atomabsorptionsspektrometrie (AAS) bestimmt werden. Hierbei wird die Gesamtmenge an Aluminium bestimmt (Aluminium gesamt).

Bevorzugt ist die in Schritt c) des erfindungsgemäßen Verfahrens verwendete Säure eine organische Säure, eine anorganische Säure oder eine Mischung davon. Die organische Säure ist bevorzugt eine Monocarbonsäure, mehr bevorzugt Ameisensäure oder Essigsäure, noch mehr bevorzugt Essigsäure. Bevorzugt ist die anorganische Säure aus der Gruppe bestehend aus Phosphorsäure, Salzsäure, Salpetersäure, Schwefelsäure oder eines ihrer Derivate ausgewählt. In einer bevorzugten Ausführungsform handelt es sich bei der anorganischen Säure um Phosphorsäure. In Schritt c) kann Salzsäure oder Schwefelsäure verwendet werden. In diesem Fall werden Mengen der Säure verwendet, die dazu führen, dass der Gehalt von Chlorid im Produkt bei höchstens 1000 ppm und/oder der Gehalt von Sulfat im Produkt bei höchstens 5000 ppm liegt.

In einer bevorzugten Ausführungsform des erfindungsgemäßen Verfahrens handelt es sich bei der in Schritt c) verwendeten Säure um eine organische Säure, bevorzugt um eine Monocarbonsäure, mehr bevorzugt um Ameisensäure oder Essigsäure, noch mehr bevorzugt um Essigsäure.

Nach dem Vermischen der wässrigen Base-Fettsäure-Aluminium-Mischung mit der Säure in Schritt c) liegt der pH-Wert der angesäuerten wässrigen Base-Fettsäure-Aluminium-Mischung im Bereich zwischen 3,5 und 7,0. Bevorzugt liegt der pH-Wert zwischen 4,5 und 7,0. Mehr bevorzugt liegt der pH-Wert zwischen 5,0 und 6,0 oder zwischen 5,3 und 5,8.

In einer Ausführungsform des erfindungsgemäßen Verfahrens werden die Fettsäure und die wässrige Lösung einer starken Base aus Schritt a) mit einer aromatischen Carbonsäure, bevorzugt Benzoesäure und/oder Derivate der Benzoesäure, vermischt. Bevorzugt erfolgt die Vermischung mit der aromatischen Carbonsäure vor der Zugabe der Aluminiumquelle zu der wässrigen Base-Fettsäure-Mischung. Mehr bevorzugt wird die aromatische Carbonsäure mit der wässrigen Lösung der starken Base vermischt, bevor die Fettsäure hinzugegeben wird. Bei den in dieser Ausführungsform gebildeten Aluminiumsalzen einer Fettsäure handelt es sich um sogenannte Aluminium-Komplexseifen. Diese enthalten neben den Fettsäureanionen auch noch Anionen der aromatischen Carbonsäuren. Die Aluminium-Komplexseifen eignen sich besonders zur Schmierfettherstellung.

Überraschenderweise hat sich gezeigt, dass Aluminiumsalze einer Fettsäure, die mit dem erfindungsgemäßen Verfahren hergestellt werden, einen niedrigen Gehalt an Natriumchlorid und Natriumsulfat, sowie anderen Salzen, aufweisen. Wird als Säure in Schritt c) des erfindungsgemäßen Verfahrens Essigsäure verwendet, so entsteht als Nebenprodukt zu den Aluminiumsalzen der Fettsäure ein Alkaliacetat, welches sich leicht auswaschen lässt. Beispielsweise weist mit dem erfindungsgemäßen Verfahren hergestelltes Aluminiumstearat einen Chloridgehalt von höchstens 1000 ppm und einen Sulfatgehalt von höchstens 5000 ppm auf und erfüllt somit die Anforderungen des Europäischen Arzneibuchs. Zudem erweist sich auch der überraschend geringe Natriumgesamtgehalt der Aluminiumsalze der Fettsäuren, welche nach den erfindungsgemäßen Verfahren erhalten werden, als vorteilhaft.

Gegenstand der Erfindung sind weiterhin Aluminiumsalze einer Fettsäure, welche erhältlich sind nach dem erfindungsgemäßen Verfahren zur Herstellung von Aluminiumsalzen einer Fettsäure. Bevorzugt haben die Aluminiumsalze einer Fettsäure einen Chloridgehalt von höchstens 1000 ppm und/oder einen Sulfatgehalt von höchstens 5000 ppm. Der Chloridgehalt wird gemäß der in der Monographie zu Aluminiumstearat genannten Vorschrift des Europäischen Arzneibuchs (European Pharmacopoeia, Edition 10.0 (2019): "Monographs A", 'Aluminium Stearate' und "Methods of Analysis", '2.4.4 Chlorides') bestimmt. Der Sulfatgehalt wird gemäß der in der Monographie zu Aluminiumstearat genannten Vorschrift des Europäischen Arzneibuchs (European Pharmacopoeia, Edition 10.0 (2019): "Monographs A", 'Aluminium Stearate' und "Methods of Analysis", '2.4.13 Sulfates') bestimmt.

Soweit nicht anders angegeben werden alle Volumina bei 23 °C gemessen. Soweit nicht anders angegeben, steht die Bezeichnung "ppm" für den Massenanteil. Ein Chloridgehalt der Aluminiumsalze einer Fettsäure von höchstens 1000 ppm bedeutet folglich, dass 1 kg Produkt höchstens 1000 mg Chlorid enthält.

Die Erfindung wird durch die nachfolgenden Beispiele näher erläutert:
Der Natriumgehalt der in den Beispielen 1 bis 5 erhaltenen Produkte wurde mittels Röntgenfluoreszenzanalyse bestimmt. Hierbei wurde die Gesamtmenge an Natrium bestimmt (Natrium gesamt).

### Beispiel V: Versuche zu Herstellung Chlorid- und Sulfat-armer Aluminiumfettsäuren (Vergleichsbeispiele)

Aluminiumstearat wurde im industriellen Maßstab durch Umsetzung von Stearinsäure mit Natronlauge und Fällung mit Aluminiumsulfat erhalten. Es ist ein weißer Feststoff, der in Wasser unlöslich ist und aufschwimmt. Das Produkt ist nach dem Abfiltrieren stark wasserhaltig.

Der Schwefelgehalt wurde durch Röntgenfluoreszenzanalyse gemessen. Wegen der Molmasse 32 g/mol für Schwefel und 96 g/mol für Sulfat ist der Sulfatgehalt dreimal höher als der Schwefelgehalt. Zusätzlich muss der Wassergehalt des Aluminumstearats berücksichtigt werden. Dieser liegt in der Größenordnung von 70 Gew-% und wurde für jede Probe gemessen.

| Probe | Behandlung | Sulfatgehalt (berechnet) [ppm] |
|---|---|---|
| Ausgangsmaterial | | 19000 |
| Ansatz 1 | 3-maliges Waschen für 900 s mit 60 Gewichtsteilen Stadtwasser, 60 °C, 10 s Anpressen des Filters | 17700 |
| Ansatz 2 | 1-maliges Waschen für 900 s mit 60 Gewichtsteilen Stadtwasser, 60 °C, 10 s Anpressen des Filters | 17700 |
| Ansatz 3 | 1-maliges Waschen für 900 s mit 60 Gewichtsteilen Stadtwasser, 20 °C, 10 s Anpressen des Filters | 19100 |
| Ansatz 4 | 1-maliges Waschen für 900 s mit 60 Gewichtsteilen VE-Wasser, 20 °C, 10 s Anpressen des Filters | 15500 |
| Ansatz 5 | 1-maliges Waschen für 600 s mit 100 Gewichtsteilen Stadtwasser, 20 °C, 10 s Anpressen des Filters | 17100 |
| Ansatz 6 | 1-maliges Waschen für 600 s mit 100 Gewichtsteilen Stadtwasser, 20 °C, ohne Anpressen des Filters | 18300 |
| Ansatz 7 | 1-maliges Waschen für 600 s mit 100 Gewichtsteilen Stadtwasser, 20 °C, ohne Anpressen des Filters, | 18300 |
| Ansatz 8 | 1-maliges Waschen für 600 s mit 100 Gewichtsteilen Stadtwasser, 20 °C, 10 s Anpressen des Filters, | 19100 |
| Ansatz 9 | 1-maliges Waschen für 1200 s mit 100 Gewichtsteilen Stadtwasser, 20 °C, 10 s Anpressen des Filters, | 19300 |

Es konnte durch Waschen unter verschiedenen Bedingungen keine hinreichende Verringerung des Sulfatgehaltes erreicht werden.

### Beispiel 1: Synthese eines Aluminium Mono-Di-Stearates

In einem Becherglas werden 1,5 L destilliertes Wasser vorgelegt und auf 80 °C erwärmt. Anschließen werden 43 g fester Natronlauge in dem Wasser gelöst. Es wird langsam durch die Zugabe von 270 g flüssiger pflanzlicher Stearinsäure (70 °C) verseift. Die Stearinsäure weist einen Gehalt von 66 Gew.-% Stearinsäure und 27 Gew.-% Palmitinsäure auf. Anschließend werden langsam 64,5 g Natriumaluminat, welches vorher in 250 g Wasser gelöst wurde, zugefügt. Nach einer Rührzeit von 15 Minuten wird mit 210 g 60%-iger Essigsäure auf einen pH-Wert von ungefähr 5,5 angesäuert. Nach weiteren 10 Minuten Rührzeit wird das Produkt über einen Büchnerfilter abfiltriert und mehrmals mit destilliertem

Wasser gewaschen. Im Trockenschrank erfolgt bei 80 °C die Trocknung auf < 1,5 % Wassergehalt. Das Produkt weist die folgenden analytischen Daten auf:

| | |
|---|---|
| • Aluminiumgehalt: | 5,6 Gew.-% |
| • freie Fettsäure: | 1,6 Gew.-% |
| • Natriumgehalt: | < 10 ppm |
| • Wassergehalt: | 0,7 Gew.-% |

### Beispiel 2: Synthese eines Aluminium Di-Tri-Stearates

In einem Becherglas werden 2 L destilliertes Wasser vorgelegt und auf 70 - 80 °C erwärmt. Anschließen werden 31,6 g fester Natronlauge in dem Wasser gelöst. Es wird langsam durch die Zugabe von 185 g flüssiger talgbasierter Stearinsäure (70 °C) verseift. Anschließend werden langsam 24,6 g Natriumaluminat, welches vorher in 180 g Wasser gelöst wurde, zugefügt. Nach einer Rührzeit von 15 Minuten wird mit 84 g 60%-iger Essigsäure auf einen pH-Wert von ungefähr 5,5 angesäuert. Nach weiteren 10 Minuten Rührzeit wird das Produkt über einen Büchnerfilter abfiltriert und mehrmals mit destilliertem Wasser gewaschen. Im Trockenschrank erfolgt bei 80 °C die Trocknung auf < 1,5 % Wassergehalt. Das Produkt weist die folgenden analytischen Daten auf:

| | |
|---|---|
| • Aluminiumgehalt: | 3,9 Gew.-% |
| • freie Fettsäure: | 12,2 Gew.-% |
| • Natriumgehalt: | < 10 ppm |
| • Wassergehalt: | 1,1 Gew.-% |

### Beispiel 3: Synthese eines Aluminium Tri-Stearates

In einem Becherglas werden 1,5 L destilliertes Wasser vorgelegt und auf 70 - 80 °C erwärmt. Anschließen werden 29,2 g fester Natronlauge in dem Wasser gelöst. Es wird langsam mit der Zugabe von 194 g flüssiger pflanzlicher Stearinsäure (70 °C) verseift. Die Stearinsäure weist einen Gehalt von 55 Gew.-% Stearinsäure und 45 Gew.-% Palmitinsäure auf. Anschließend werden langsam 21,2 g Natriumaluminat, welches vorher in 250 g Wasser gelöst wurde, zugefügt. Nach einer Rührzeit von 15 Minuten wird mit 73 g 60%-iger Essigsäure auf einen pH-Wert von ungefähr 5,5 angesäuert. Nach weiteren 10 Minuten Rührzeit wird das Produkt über einen Büchnerfilter abfiltriert und mehrmals mit destilliertem Wasser gewaschen. Im Trockenschrank erfolgt bei 80 °C die Trocknung auf < 1,5 % Wassergehalt. Das Produkt weist die folgenden analytischen Daten auf:

| | |
|---|---|
| • Aluminiumgehalt: | 3,4 Gew.-% |
| • freie Fettsäure: | 18,3 Gew.-% |
| • Natriumgehalt: | < 10 ppm |
| • Wassergehalt: | 0,8 Gew.-% |

### Beispiel 4: Synthese eines Aluminium Mono-Di-Stearates

In einem Becherglas werden 1,5 L destilliertes Wasser vorgelegt und auf 80 °C erwärmt. Anschließen werden 43 g fester Natronlauge in dem Wasser gelöst. Es wird langsam durch die Zugabe von 270 g flüssiger pflanzlicher Stearinsäure (70 °C) verseift. Die Stearinsäure weist einen Gehalt von 66 Gew.-% Stearinsäure und 27 Gew.-% Palmitinsäure auf. Anschließend werden langsam 64,5 g Natriumaluminat, welches vorher in 250 g Wasser gelöst wurde, zugefügt. Nach einer Rührzeit von 15 Minuten wird mit 82,3 g 85%-iger Phosphorsäure auf einen pH-Wert von ungefähr 5,5 angesäuert. Nach weiteren 10 Minuten Rührzeit wird das Produkt über einen Büchnerfilter abfiltriert und mehrmals mit destilliertem Wasser gewaschen. Im Trockenschrank erfolgt bei 80 °C die Trocknung auf < 1,5 % Wassergehalt. Das Produkt weist die folgenden analytischen Daten auf:

| | |
|---|---|
| • Aluminiumgehalt: | 5,2 Gew.-% |
| • freie Fettsäure: | 2,2 Gew.-% |
| • Natriumgehalt: | < 10 ppm |
| • Wassergehalt: | 1,2 Gew.-% |

### Beispiel 5: Synthese einer Aluminium-Komplexseife

In einem Becherglas werden 1,5 L destilliertes Wasser vorgelegt und auf 80 °C erwärmt. Anschließen werden 43 g fester Natronlauge in dem Wasser gelöst. Es wird langsam durch die Zugabe von 26,8 g fester Benzoesäure verseift. Anschließend erfolgt die Zugabe von 210 g flüssiger pflanzlicher Stearinsäure. Anschließend werden langsam 64,5 g Natriumaluminat, welches vorher in 250 g Wasser gelöst wurde, zugefügt. Nach einer Rührzeit von 15 Minuten wird mit 210 g 60%-iger Essigsäure auf einen pH-Wert von ungefähr 5,5 angesäuert. Nach weiteren 10 Minuten Rührzeit wird das Produkt über einen Büchnerfilter abfiltriert und mehrmals mit destilliertem Wasser gewaschen. Im Trockenschrank erfolgt bei 80 °C die Trocknung auf < 1,5 % Wassergehalt. Das Produkt weist die folgenden analytischen Daten auf:

| | |
|---|---|
| • Aluminiumgehalt: | 5,3 Gew.-% |
| • freie Fettsäure: | 3,2 Gew.-% |
| • Natriumgehalt: | < 10 ppm |
| • Wassergehalt: | 1,2 Gew.-% |

## Patentansprüche

1. Verfahren zur Herstellung von Aluminiumsalzen einer Fettsäure, umfassend die folgenden Schritte:
a) Vermischen einer Fettsäure und einer wässrigen Lösung einer starken Base zur Herstellung einer wässrigen Base-Fettsäure-Mischung,
b) Vermischen der wässrigen Base-Fettsäure-Mischung mit einer Aluminiumquelle zur Herstellung einer wässrigen Base-Fettsäure-Aluminium-Mischung,
c) Vermischen der wässrigen Base-Fettsäure-Aluminium-Mischung mit einer Säure, und
d) Abtrennung der entstandenen Aluminiumsalze der Fettsäure.

2. Verfahren nach Anspruch 1, wobei die Aluminiumsalze einer Fettsäure Mono-, Di- oder Trifettsäurealuminiumsalze sind.

3. Verfahren nach einem der Ansprüche 1 oder 2, wobei die Fettsäure eine Fettsäure der Kettenlänge C6 bis C28 oder eine Mischung von Fettsäuren, bevorzugt Stearinsäure, Palmitinsäure oder eine Mischung dieser Fettsäuren, ist.

4. Verfahren nach einem der Ansprüche 1 bis 3, wobei die Fettsäure Stearinsäure ist.

5. Verfahren nach einem der Ansprüche 1 bis 4, wobei die starke Base ein Alkalihydroxid, bevorzugt Natriumhydroxid oder Kaliumhydroxid, besonders bevorzugt Natriumhydroxid, ist.

6. Verfahren nach einem der Ansprüche 1 bis 5, wobei die Aluminiumquelle ein Alkalialuminat oder Aluminiumpulver, bevorzugt Natriumaluminat oder Aluminiumpulver, besonders bevorzugt Natriumaluminat, ist.

7. Verfahren nach einem der Ansprüche 1 bis 6, wobei die in Schritt c) verwendete Säure eine organische Säure, eine anorganische Säure oder eine Mischung aus beiden ist.

8. Verfahren nach Anspruch 7, wobei die organische Säure bevorzugt eine Monocarbonsäure, besonders bevorzugt Ameisensäure oder Essigsäure, ist.

9. Verfahren nach einem der Ansprüche 7 oder 8, wobei die anorganische Säure bevorzugt Phosphorsäure, Salzsäure, Salpetersäure, Schwefelsäure oder eines ihrer Derivate, besonders bevorzugt Phosphorsäure, ist.

10. Verfahren nach einem der Ansprüche 1 bis 9, wobei die Abtrennung der entstandenen Aluminiumsalze der Fettsäure durch ein physikalisches Trennverfahren, bevorzugt durch Filtration, erfolgt.

11. Verfahren nach Anspruch 1, wobei die Fettsäure und die wässrige Lösung einer starken Base mit einer aromatischen Carbonsäure, bevorzugt Benzoesäure, vermischt werden.

12. Verfahren nach einem der Ansprüche 1 bis 11, wobei der pH-Wert der wässrigen Base-Fettsäure-Mischung im Bereich zwischen 7 und 12 liegt.

13. Verfahren nach einem der Ansprüche 1 bis 12, wobei der pH-Wert der angesäuerten wässrigen Base-Fettsäure-Aluminium-Mischung im Bereich zwischen 3,5 und 7 liegt.

14. Aluminiumsalze einer Fettsäure erhältlich nach einem Verfahren nach einem der Ansprüche 1 bis 13, wobei die Aluminiumsalze einer Fettsäure einen Gehalt von höchstens 1000 ppm Chlorid und 5000 ppm Sulfat haben.

## Claims

1. A process for the production of aluminum salts of a fatty acid, comprising the following steps:
a) mixing a fatty acid and an aqueous solution of a strong base to prepare an aqueous base/fatty acid mixture,
b) mixing said aqueous base/fatty acid mixture with an aluminum source to prepare an aqueous base/fatty acid/aluminum mixture,
c) mixing said aqueous base/fatty acid/aluminum mixture with an acid, and
d) separating the produced aluminum salts of the fatty acid.

2. The process according to claim 1, wherein said aluminum salts of a fatty acid are mono-, di- or tri-fatty acid aluminum salts.

3. The process according to either of claims 1 or 2, wherein said fatty acid is a fatty acid having a chain length of C6 to C28, or a mixture of fatty acids, preferably stearic acid, palmitic acid, or a mixture of such fatty acids.

4. The process according to any of claims 1 to 3, wherein said fatty acid is stearic acid.

5. The process according to any of claims 1 to 4, wherein said strong base is an alkali hydroxide, preferably sodium hydroxide or potassium hydroxide, more preferably sodium hydroxide.

6. The process according to any of claims 1 to 5, wherein said aluminum source is an alkali aluminate or aluminum powder, preferably sodium aluminate or aluminum powder, more preferably sodium aluminate.

7. The process according to any of claims 1 to 6, wherein the acid used in step c) is an organic acid, an inorganic acid, or a mixture of both.

8. The process according to claim 7, wherein said organic acid preferably is a monocarboxylic acid, more preferably formic acid or acetic acid.

9. The process according to either of claims 7 or 8, wherein said inorganic acid is preferably phosphoric acid, hydrochloric acid, nitric acid, sulfuric acid, or any of their derivatives, more preferably phosphoric acid.

10. The process according to any of claims 1 to 9, wherein the separating of the produced aluminum salts of the fatty acid is effected by a physical separation method, preferably by filtration.

11. The process according to claim 1, wherein the fatty acid and the aqueous solution of a strong base are mixed with an aromatic carboxylic acid, preferably benzoic acid.

12. The process according to any of claims 1 to 11, wherein the pH value of said aqueous base/fatty acid mixture is within a range of from 7 to 12.

13. The process according to any of claims 1 to 12, wherein the pH value of said acidified aqueous base/fatty acid/aluminum mixture is within a range of from 3.5 to 7.

14. Aluminum salts of a fatty acid obtainable by a process according to any of claims 1 to 13, wherein the aluminum salts of a fatty acid have a content of at most 1000 ppm chloride and 5000 ppm sulfate.

## Revendications

1. Procédé de préparation de sels d'aluminium d'un acide gras, comprenant les étapes suivantes :
a) mélange d'un acide gras et d'une solution aqueuse d'une base forte pour la préparation d'un mélange aqueux base-acide gras,
b) mélange du mélange aqueux base-acide gras avec une source d'aluminium pour la préparation d'un mélange aqueux base-acide gras-aluminium,
c) mélange du mélange aqueux base-acide gras-aluminium avec un acide ; et
d) séparation des sels d'aluminium résultants de l'acide gras.

2. Procédé selon la revendication 1, dans lequel les sels d'aluminium d'un acide gras sont des sels d'aluminium de monoacide gras, diacide gras ou triacide gras.

3. Procédé selon l'une des revendications 1 ou 2, dans lequel l'acide gras est un acide gras de longueur de chaîne C6 à C28 ou un mélange d'acides gras, de préférence d'acide stéarique, acide palmitique ou un mélange desdits acides gras.

4. Procédé selon l'une des revendications 1 à 3, dans lequel l'acide gras est un acide stéarique.

5. Procédé selon l'une des revendications 1 à 4, dans lequel la base forte est un hydroxyde alcalin, de préférence un hydroxyde de sodium ou un hydroxyde de potassium, de manière particulièrement préférée un hydroxyde de sodium.

6. Procédé selon l'une des revendications 1 à 5, dans lequel la source d'aluminium est un aluminate alcalin ou une poudre d'aluminium, de préférence un aluminate de sodium ou une poudre d'aluminium, de manière particulièrement préférée un aluminate de sodium.

7. Procédé selon l'une des revendications 1 à 6, dans lequel l'acide utilisé à l'étape c) est un acide organique, un acide inorganique ou un mélange des deux.

8. Procédé selon la revendication 7, dans lequel l'acide organique est de préférence un acide monocarboxylique, de manière particulièrement préférée un acide formique ou un acide acétique.

9. Procédé selon l'une des revendications 7 ou 8, dans lequel l'acide inorganique est de préférence un acide phosphorique, acide chlorhydrique, acide nitrique, acide sulfurique ou l'un de leurs dérivés, de manière particulièrement préférée un acide phosphorique.

10. Procédé selon l'une des revendications 1 à 9, dans lequel la séparation des sels d'aluminium résultants de l'acide gras est effectuée par un procédé de séparation physique, de préférence par filtration.

11. Procédé selon la revendication 1, dans lequel l'acide gras et la solution aqueuse d'une base forte sont mélangés avec un acide carboxylique aromatique, de préférence un acide benzoïque.

12. Procédé selon l'une des revendications 1 à 11, dans lequel la valeur de pH du mélange aqueux base-acide gras est dans une plage comprise entre 7 et 12.

13. Procédé selon l'une des revendications 1 à 12, dans lequel la valeur de pH du mélange aqueux base-acide gras-aluminium acidifié est dans une plage comprise entre 3,5 et 7.

14. Sels d'aluminium d'un acide gras pouvant être obtenus selon un procédé selon l'une des revendications 1 à 13, dans lesquels les sels d'aluminium d'un acide gras présentent une teneur d'au plus 1000 ppm de chlorure et 5000 ppm de sulfate.
